(19) Europäisches Patentamt
European Patent Office
Office européen des brevets

(11) Veröffentlichungsnummer: **0 001 602**
**A1**

# (12) EUROPÄISCHE PATENTANMELDUNG

(21) Anmeldenummer: **78101097.0**

(22) Anmeldetag: **07.10.78**

(51) Int. Cl.$^2$: **C 07 F 9/65**
A 01 N 9/36

(30) Priorität: **21.10.77 DE 2747357**

(43) Veröffentlichungstag der Anmeldung:
**02.05.79 Patentblatt 79/9**

(84) Benannte Vertragsstaaten:
**BE CH DE FR GB NL**

(71) Anmelder: **Bayer Aktiengesellschaft**
**Zentralbereich Patente,Marken und Lizenzen Bayerwerk**
**D-5090 Leverkusen 1(DE)**

(72) Erfinder: **Maurer,Fritz,Dr.**
**Roeberstrasse 8**
**D-5600 Wuppertal 1(DE)**

(72) Erfinder: **Schröder, Rolf, Dr.**
**Pahlkestrasse 17**
**D-5600 Wuppertal 1(DE)**

(72) Erfinder: **Hammann, Ingeborg, Dr.**
**Belfortstrasse 9**
**D-5000 Köln 1(DE)**

(72) Erfinder: **Behrenz, Wolfgang, Dr.**
**Untergruendemich 14**
**D-5063 Overath(DE)**

(54) **Substituierte Pyrimidinyl(thiono) (thiol)phosphor(phosphon) -säureester bzw. -esteramide, Verfahren zu ihrer Herstellung und ihre Verwendung als Insektizide und Akarizide.**

(57) Die Erfindung betrifft neue substituierte Pyrimidinyl (thiono)-(thiol)- phosphor (phosphon)-säureester bzw. esteramide der Formel

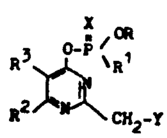

in welcher

| | |
|---|---|
| R | für Alkyl, |
| $R^1$ | für Alkyl, Phenyl, Alkoxy, Alkylthio oder Alkylamino, |
| $R^2$ und $R^3$ | gleich oder verschieden sind und für Wasserstoff, Halogen oder Alkyl, |
| Y | für Alkoxy, Alkylthio oder Mono-bzw. Dialkylamino und |
| X | für Sauerstoff oder Schwefel stehen. |

Diese neuen Verbindungen besitzen eine ausgezeichnete insektizide und akarizide Wirkung.

EP 0 001 602 A1

Croydon Printing Company Ltd.

BAYER AKTIENGESELLSCHAFT 5090 Leverkusen, Bayerwerk
Zentralbereich Ad/AB
Patente, Marken und Lizenzen

Substituierte Pyrimidinyl(thiono)(thiol)phosphor(phosphon)-säureester bzw. -esteramide, Verfahren zu ihrer Herstellung und ihre Verwendung als Insektizide und Akarizide

Die vorliegende Erfindung betrifft neue substituierte Pyrimidinyl(thiono)(thiol)-phosphor(phosphon)-säureester bzw. -esteramide, mehrere Verfahren zu ihrer Herstellung und ihre Verwendung als Insektizide und Akarizide.

Es ist bereits bekannt, daß O,O-Dialkyl- bzw. O,S-Dialkyl-O-pyrimidinylthionophosphorsäureester, z.B. O,O-Diäthyl-O-[2-methylthio-6-methyl-pyrimidin(4)yl]-thiono- bzw. O-Äthyl-S-n-propyl-O-[2-isopropyl-6-methyl-pyrimidin(4)yl]-thionothiolphosphorsäureester, insektizide und akarizide Eigenschaften haben (vergleiche US-Patentschrift 2 754 243 und Deutsche Offenlegungsschrift 2 36o 877).

Es wurden nun die neuen substituierten Pyrimidinyl(thiono)-(thiol)-phosphor(phosphon)-säureester bzw. -esteramide der Formel

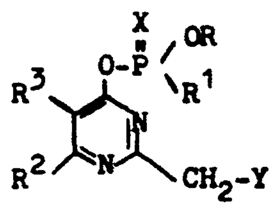

(I)

in welcher

R für Alkyl,

$R^1$ für Alkyl, Phenyl, Alkoxy, Alkylthio oder Alkylamino,

$R^2$ und $R^3$ gleich oder verschieden sind und für Wasserstoff, Halogen oder Alkyl,

Y für Alkoxy, Alkylthio oder Mono- bzw. Dialkylamino und

X für Sauerstoff oder Schwefel stehen,

synthetisiert.

Diese neuen Verbindungen besitzen eine ausgezeichnete insektizide und akarizide Wirkung.

Weiterhin wurde gefunden, daß man die substituierten Pyrimidinyl-(thiono)(thiol)-phosphor(phosphon)-säureester bzw. -esteramide der Formel (I) erhält, wenn man

a) (Thiono)(Thiol)Phosphor(phosphon)-säureester- bzw. -esteramidhalogenide der Formel

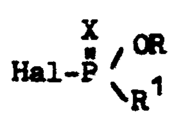

(II)

in welcher

R, $R^1$ und X die oben angegebene Bedeutung haben und

Hal für Halogen, vorzugsweise Chlor, steht, mit substituierten 4-Hydroxy-pyrimidinen der Formel

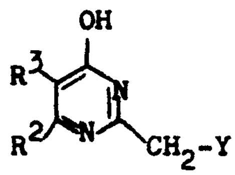

(III)

in welcher

$R^2$, $R^3$ und Y die oben angegebene Bedeutung haben, gegebenenfalls in Gegenwart eines Säureakzeptors oder gegebenenfalls in Form der Alkali-, Erdalkali- oder Ammoniumsalze und gegebenenfalls in Gegenwart eines Lösungsmittels umsetzt, oder für den Fall, daß Y für Mono- bzw. Dialkylamino steht,

wenn man

b) 2-Halogenmethyl-pyrimidin(4)yl(thiono)(thiol)-phosphor-(phosphon)-säureester bzw. -esteramide der Formel

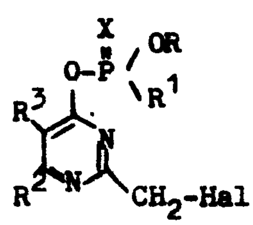

(IV)

in welcher

R bis $R^3$, X und Hal die oben angegebene Bedeutung haben,

mit Verbindungen der Formel

Y'H (V)

in welcher

Y' für Mono- oder Dialkylamino steht,

gegebenenfalls in Gegenwart eines Säureakzeptors und gegebenenfalls in Gegenwart eines Lösungsmittels umsetzt.

Überraschenderweise zeigen die erfindungsgemäßen substituierten Pyrimidinyl(thiono)(thiol)-phosphor(phosphon)-säureester bzw. -esteramide eine bessere insektizide und akarizide Wirkung als die vorbekannten Verbindungen analoger Konstitution und gleicher Wirkungsrichtung. Die Verbindungen vorliegender Erfindung stellen somit eine echte Bereicherung der Technik dar.

Verwendet man beispielsweise nach Verfahrensvariante a) O-Äthyl-S-n-propyl-thionothiolphosphorsäurediesterchlorid und 2-Methoxymethyl-4-hydroxy-5,6-dimethylpyrimidin und nach Verfahrensvariante b) O-Äthyl-S-n-propyl-O-[2-chlormethyl-pyrimidin(4)yl]-thionothiolphosphorsäuretriester und Diäthylamin-Hydrochlorid als Ausgangsmaterialien, so kann der Reaktionsablauf durch die folgenden Formelschemata wiedergegeben werden:

a) 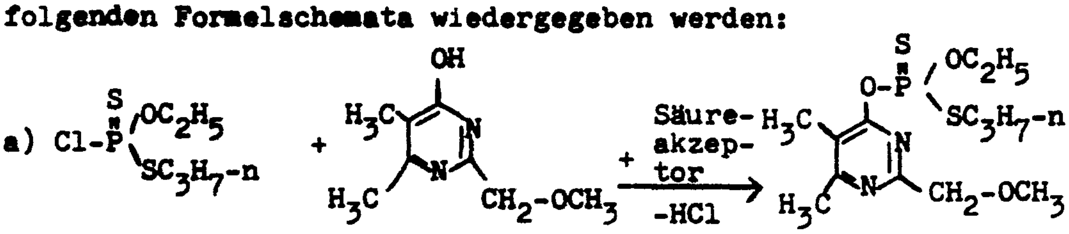

b) 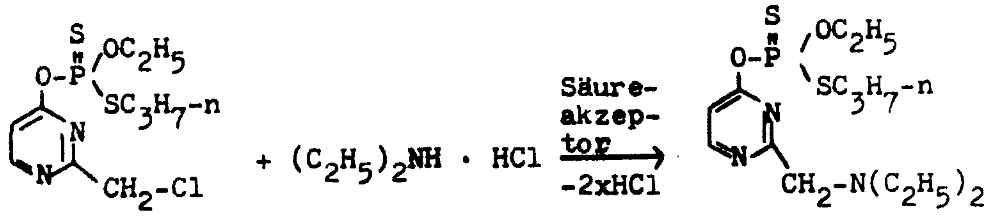


Die zu verwendenden Ausgangsstoffe sind durch die Formeln (II) bis (V) allgemein definiert. Vorzugsweise stehen darin jedoch

R für geradkettiges oder verzweigtes Alkyl mit 1 bis 6, vorzugsweise 1 bis 4, Kohlenstoffatomen,

$R^1$ für geradkettiges oder verzweigtes Alkyl oder Alkoxy mit 1 bis 6, vorzugsweise 1 bis 3, Kohlenstoffatomen, für Alkylthio bzw. Monoalkylamino mit 1 bis 6, vorzugsweise 1 bis 4, Kohlenstoffatomen oder Phenyl,

$R^2$ für Wasserstoff, Methyl oder Äthyl,

$R^3$ für Wasserstoff, Chlor, Brom oder Methyl,

Y für geradkettiges oder verzweigtes Alkoxy bzw. Alkylthio oder Monoalkylamino mit 1 bis 6, vorzugsweise 1 bis 4, Kohlenstoffatomen je Alkylrest oder Dialkylamino mit 1 bis 5, vorzugsweise 1 oder 2, Kohlenstoffatomen je Alkylrest,

Y' für Mono- bzw. Dialkylamino der unter Y angegebenen Bedeutung und

X für Schwefel.

Die als Ausgangsstoffe zu verwendenden (Thiono)(Thiol)Phosphor(phosphon)-säureester- bzw. -esteramidhalogenide (II) sind bekannt und nach literaturbekannten Verfahren auch technisch gut herstellbar. Als Beispiele dafür seien im einzelnen genannt:

O,O-Dimethyl-, O,O-Diäthyl-, O,O-Di-n-propyl-, O,O-Di-iso-propyl-, O-Methyl-O-äthyl-, O-Methyl-O-n-propyl-, O-Methyl-O-iso-propyl-, O-Methyl-O-n-butyl-, O-Methyl-O-iso-butyl-, O-Methyl-O-sek.-butyl-, O-Äthyl-O-n-propyl-, O-Äthyl-O-iso-propyl-, O-Äthyl-O-n-butyl-, O-Äthyl-O-sek.-butyl-, O-Äthyl-O-iso-butyl-, O-n-Propyl-O-butyl- bzw. O-iso-Propyl-O-butylphosphorsäurediesterchlorid und die entsprechenden Thionoanalogen, ferner

O,S-Dimethyl-, O,S-Diäthyl-, O,S-Di-n-propyl-, O,S-Di-iso-propyl-, O,S-Di-n-butyl-, O,S-Di-iso-butyl-, O,S-Di-sek.-butyl-, O-Äthyl-S-n-propyl-, O-Äthyl-S-iso-propyl-, O-Äthyl-S-n-butyl-, O-Äthyl-S-sek.-butyl-, O-n-Propyl-S-äthyl-, O-n-Propyl-S-iso-propyl-, O-n-Butyl-S-n-propyl- und O-sek.-Butyl-S-äthylthiolphosphorsäurediesterhalogenide und die entsprechenden Thionoanalogen, ferner

O-Methyl-N-methyl-, O-Methyl-N-äthyl-, O-Methyl-N-n-propyl-, O-Methyl-N-iso-propyl-, O-Äthyl-N-methyl-, O-Äthyl-N-äthyl-, O-Äthyl-N-n-propyl-, O-Äthyl-N-iso-propyl-, O-n-Propyl-N-methyl-, O-n-Propyl-N-äthyl-, O-n-Propyl-N-n-propyl-, O-n-Propyl-N-iso-propyl-, O-iso-Propyl-N-methyl-, O-iso-Propyl-N-äthyl-, O-iso-Propyl-N-n-propyl-, O-iso-Propyl-N-iso-propyl-, O-n-Butyl-N-methyl-, O-n-Butyl-N-äthyl-, O-n-Butyl-N-äthyl-, O-n-Butyl-N-n-propyl-, O-n-Butyl-N-iso-propyl-, O-iso-Butyl-N-methyl-, O-iso-Butyl-N-äthyl-, O-iso-Butyl-N-n-propyl-, O-iso-Butyl-N-iso-propyl-, O-sek.-Butyl-N-methyl-, O-sek.-Butyl-N-äthyl-, O-sek.-Butyl-N-n-propyl- und O-sek.-Butyl-N-iso-propyl-phosphorsäuremonoesteramidchlorid und die entsprechenden Thionoanalogen und

O-Methyl-, O-Äthyl-, O-n-Propyl-, O-iso-Propyl-, O-n-Butyl-,

O-iso-Butyl- und O-sek.-Butylmethan- bzw. -äthan-, -n-propan-, -iso-propan-, -n-butan-, -iso-butan-, -sek.-butan- bzw. -phenylphosphonsäureesterhalogenide und die entsprechenden Thionoanalogen.

Die weiterhin als Ausgangsstoffe zu verwendenden substituierten 4-Hydroxy-pyrimidine (III) sind teilweise bekannt oder können aus den bekannten 2-Halogen-methyl-4-hydroxy-pyrimidinen (vergleiche Verfahren b) durch Umsetzung mit Alkoholen, Mercaptanen oder Aminen gewonnen werden. Als Beispiele dafür seien im einzelnen genannt: 2-Methoxymethyl-, 2-Äthoxymethyl-, 2-n-Propoxymethyl-, 2-iso-Propoxymethyl-, 2-n-Butoxymethyl-, 2-iso-Butoxymethyl-, 2-sek.-Butoxymethyl-, 2-tert.-Butoxymethyl-, 2-Methylthiomethyl-, 2-Äthylthiomethyl-, 2-n-Propylthiomethyl-, 2-iso-Propylthiomethyl-, 2-n-Butylthiomethyl-, 2-iso-Butylthiomethyl-, 2-sek.-Butylthiomethyl-, 2-tert.-Butylthiomethyl-, 2-Monomethylaminomethyl-, 2-Monoäthylaminomethyl-, 2-Mono-n-propylaminomethyl-, 2-Mono-iso-propylaminomethyl-, 2-Mono-n-butylaminomethyl-, 2-Mono-iso-butylaminomethyl-, 2-Mono-sek.-butylaminomethyl-, 2-Mono-tert.-butylaminomethyl-, 2-Dimethylaminomethyl-, 2-Diäthylaminomethyl-, 2-Methoxymethyl-5-chlor-, 2-Äthoxymethyl-5-chlor-, 2-n-Propoxymethyl-5-chlor-, 2-iso-Propoxymethyl-5-chlor-, 2-n-Butoxymethyl-5-chlor-, 2-iso-Butoxymethyl-5-chlor-, 2-sek.-Butoxymethyl-5-chlor-, 2-tert.-Butoxymethyl-5-chlor-, 2-Methylthiomethyl-5-chlor-, 2-Äthylthiomethyl-5-chlor-, 2-n-Propylthiomethyl-5-chlor-, 2-iso-Propylthiomethyl-5-chlor-, 2-n-Butylthiomethyl-5-chlor-, 2-iso-Butylthiomethyl-5-chlor-, 2-sek.-Butylthiomethyl-5-chlor-, 2-tert.-Butylthiomethyl-5-chlor-, 2-Monomethylaminomethyl-5-chlor-, 2-Monoäthylaminomethyl-5-chlor-, 2-Mono-n-propylaminomethyl-5-chlor-, 2-Mono-iso-propylaminomethyl-5-chlor-, 2-Mono-iso-butylaminomethyl—5-chlor-, 2-Mono-sek.-butylaminomethyl-5-chlor-, 2-Mono-tert.-butylaminomethyl-5-chlor-, 2-Dimethylaminomethyl-5-chlor-, 2-Diäthylaminomethyl-5-chlor-, 2-Methoxymethyl-5-brom-, 2-Äthoxymethyl-5-brom-, 2-n-Propoxymethyl-5-brom-, 2-iso-Propoxymethyl-5-brom-, 2-n-Butoxymethyl-5-brom-, 2-iso-Butoxymethyl-5-brom-, 2-sek.-Butoxymethyl-5-brom-, 2-tert.-Butoxymethyl-5-brom-, 2-Methylthiomethyl-5-brom-, 2-Äthylthiomethyl-5-brom-, 2-n-Propylthiomethyl-5-brom-, 2-iso-Propylthiomethyl-5-brom-, 2-n-Butylthiomethyl-5-brom-, 2-iso-Butylthiomethyl-5-brom-, 2-sek.-Butylthiomethyl-5-brom-, 2-tert.-Butylthiomethyl-5-brom-, 2-Monomethylaminomethyl-5-brom-, 2-Monoäthylaminomethyl-5-brom-, 2-Mono-n-propylaminomethyl-5-brom-, 2-Mono-iso-propylaminomethyl-5-brom-, 2-Mono-n-butylaminomethyl-5-brom-, 2-Mono-iso-butylaminomethyl-5-brom-, 2-Mono-sek.-butylaminomethyl-5-brom-, 2-Mono-tert.-butylaminomethyl-5-brom-, 2-Dimethylaminomethyl-5-brom-, 2-Diäthylaminomethyl-5-brom-, 2-Methoxymethyl-5-methyl-, 2-Äthoxymethyl-5-methyl-, 2-n-Propoxymethyl-5-methyl-, 2-iso-Propoxymethyl-5-methyl-, 2-n-Butoxymethyl-5-methyl-, 2-iso-Butoxymethyl-5-methyl-, 2-sek.-Butoxymethyl-5-methyl-, 2-tert.-Butoxymethyl-5-methyl-, 2-Methylthiomethyl-5-methyl-, 2-Äthylthiomethyl-5-methyl-, 2-n-Propylthiomethyl-5-methyl-, 2-iso-Propylthiomethyl-5-methyl-, 2-n-Butylthiomethyl-5-methyl-, 2-iso-Butylthiomethyl-5-methyl-, 2-sek.-Butylthiomethyl-5-methyl-, 2-tert.-Butylthiomethyl-5-methyl-, 2-Monomethylaminomethyl-5-methyl-, 2-Monoäthylaminomethyl-5-methyl-, 2-Mono-n-propylaminomethyl-5-methyl-, 2-Mono-iso-propylaminomethyl-5-methyl-, 2-Mono-n-butylaminomethyl-5-methyl-, 2-Mono-iso-butylaminomethyl-5-methyl-, 2-Mono-sek.-butylaminomethyl-5-methyl-, 2-Mono-tert.-butylaminomethyl-5-methyl-, 2-Dimethylaminomethyl-5-methyl-, 2-Diäthylaminomethyl-5-methyl-, 2-Methoxymethyl-5-chlor-6-methyl-, 2-Äthoxymethyl-5-chlor-6-methyl-, 2-n-Propoxymethyl-5-chlor-6-methyl-, 2-iso-Propoxymethyl-5-chlor-6-methyl-, 2-n-Butoxymethyl-5-chlor-6-methyl-, 2-iso-Butoxymethyl-5-chlor-6-methyl-, 2-sek.-Butoxymethyl-5-chlor-6-methyl-, 2-tert.-

Butoxymethyl-5-chlor-6-methyl-, 2-Methylthiomethyl-5-chlor-6-methyl-, 2-Äthylthiomethyl-5-chlor-6-methyl-, 2-n-Propylthiomethyl-5-chlor-6-methyl-, 2-iso-Propylthiomethyl-5-chlor-6-methyl-, 2-n-Butylthiomethyl-5-chlor-6-methyl-, 2-iso-Butylthiomethyl-5-chlor-6-methyl-, 2-sek.-Butylthiomethyl-5-chlor-6-methyl-, 2-tert.-Butylthiomethyl-5-chlor-6-methyl-, 2-Monomethylaminomethyl-5-chlor-6-methyl-, 2-Monoäthylaminomethyl-5-chlor-6-methyl-, 2-Mono-n-propylaminomethyl-5-chlor-6-methyl-, 2-Mono-iso-propylaminomethyl-5-chlor-6-methyl-, 2-Mono-n-butylaminomethyl-5-chlor-6-methyl-, 2-Mono-iso-butylaminomethyl-5-chlor-6-methyl-, 2-Mono-sek.-butylaminomethyl-5-chlor-6-methyl-, 2-Mono-tert.-butylaminomethyl-5-chlor-6-methyl-, 2-Dimethylaminomethyl-5-chlor-6-methyl-, 2-Diäthylaminomethyl-5-chlor-6-methyl-, 2-Methoxymethyl-5-brom-6-methyl-, 2-Äthoxymethyl-5-brom-6-methyl-, 2-n-Propoxymethyl-5-brom-6-methyl-, 2-iso-Propoxymethyl-5-brom-6-methyl-, 2-n-Butoxymethyl-5-brom-6-methyl-, 2-iso-Butoxymethyl-5-brom-6-methyl-, 2-sek.-Butoxymethyl-5-brom-6-methyl-, 2-tert.-Butoxymethyl-5-brom-6-methyl-, 2-Methylthiomethyl-5-brom-6-methyl-, 2-Äthylthiomethyl-5-brom-6-methyl-, 2-n-Propylthiomethyl-5-brom-6-methyl-, 2-iso-Propylthiomethyl-5-brom-6-methyl-, 2-n-Butylthiomethyl-5-brom-6-methyl-, 2-iso-Butylthiomethyl-5-brom-6-methyl-, 2-sek.-Butylthiomethyl-5-brom-6-methyl-, 2-tert.-Butylthiomethyl-5-brom-6-methyl-, 2-Monomethylaminomethyl-5-brom-6-methyl-, 2-Monoäthylaminomethyl-5-brom-6-methyl-, 2-Mono-n-propylaminomethyl-5-brom-6-methyl-, 2-Mono-iso-propylaminomethyl-5-brom-6-methyl-, 2-Mono-n-butylaminomethyl-5-brom-6-methyl-, 2-Mono-iso-butylaminomethyl-5-brom-6-methyl-, 2-Mono-sek.-butylaminomethyl-5-brom-6-methyl-, 2-Mono-tert.-butylaminomethyl-5-brom-6-methyl-, 2-Dimethylaminomethyl-5-brom-6-methyl-, 2-Diäthylaminomethyl-5-brom-6-methyl-, 2-Methoxymethyl-5,6-dimethyl-, 2-Äthoxymethyl-5,6-dimethyl-, 2-n-Propoxymethyl-5,6-dimethyl-, 2-iso-Propoxymethyl-5,6-dimethyl-, 2-n-Butoxymethyl-5,6-dimethyl-,

2-iso-Butoxymethyl-5,6-dimethyl-, 2-sek.-Butoxymethyl-5,6-dimethyl-, 2-tert.-Butoxymethyl-5,6-dimethyl-, 2-Methylthiomethyl-5,6-dimethyl-, 2-Äthylthiomethyl-5,6-dimethyl-, 2-n-Propylthiomethyl-5,6-dimethyl-, 2-iso-Propylthiomethyl-5,6-dimethyl-, 2-n-Butylthiomethyl-5,6-dimethyl-, 2-iso-Butylthiomethyl-5,6-dimethyl-, 2-sek.-Butylthiomethyl-5,6-dimethyl-, 2-tert.-Butylthiomethyl-5,6-dimethyl-, 2-Monomethylaminomethyl-5,6-dimethyl-, 2-Monoäthylaminomethyl-5,6-dimethyl-, 2-Mono-n-propylaminomethyl-5,6-dimethyl-, 2-Mono-iso-propylaminomethyl-5,6-dimethyl-, 2-Mono-n-butylaminomethyl-5,6-dimethyl-, 2-Mono-iso-butylaminomethyl-5,6-dimethyl-, 2-Mono-sek.-butylaminomethyl-5,6-dimethyl-, 2-Mono-tert.-butylaminomethyl-5,6-dimethyl-, 2-Dimethylaminomethyl-5,6-dimethyl-, 2-Diäthylaminomethyl-5,6-dimethyl-, 2-Methoxymethyl-6-methyl-, 2-Äthoxymethyl-6-methyl-, 2-n-Propoxymethyl-6-methyl-, 2-iso-Propoxymethyl-6-methyl-, 2-n-Butoxymethyl-6-methyl-, 2-iso-Butoxymethyl-6-methyl-, 2-sek.-Butoxymethyl-6-methyl-, 2-tert.-Butoxymethyl-6-methyl-, 2-Methylthiomethyl-6-methyl-, 2-Äthylthiomethyl-6-methyl-, 2-n-Propylthiomethyl-6-methyl-, 2-iso-Propylthiomethyl-6-methyl-, 2-n-Butylthiomethyl-6-methyl-, 2-iso-Butylthiomethyl-6-methyl-, 2-sek.-Butylthiomethyl-6-methyl-, 2-tert.-Butylthiomethyl-6-methyl-, 2-Monomethylaminomethyl-6-methyl-, 2-Monoäthylaminomethyl-6-methyl-, 2-Mono-n-propylaminomethyl-6-methyl-, 2-Mono-iso-propylaminomethyl-6-methyl-, 2-Mono-n-butylaminomethyl-6-methyl-, 2-Mono-iso-butylaminobutyl-6-methyl-, 2-Mono-sek.-butylaminomethyl-6-methyl-, 2-Mono-tert.-butylaminomethyl-6-methyl-, 2-Dimethylaminomethyl-6-methyl-, 2-Diäthylaminomethyl-6-methyl-, 2-Methoxymethyl-6-äthyl-, 2-Äthoxymethyl-6-äthyl-, 2-n-Propoxymethyl-6-äthyl-, 2-iso-Propoxymethyl-6-äthyl-, 2-n-Butoxymethyl-6-äthyl-, 2-iso-Butoxymethyl-6-äthyl-, 2-sek.-Butoxymethyl-6-äthyl-, 2-tert.-Butoxymethyl-6-äthyl-, 2-Methylthiomethyl-6-äthyl-, 2-Äthylthiomethyl-6-äthyl-, 2-n-Propylthiomethyl-6-äthyl-, 2-iso-Propylthiomethyl-6-äthyl-, 2-n-Butylthiomethyl-6-äthyl-,

2-iso-Butylthiomethyl-6-äthyl-, 2-sek.-Butylthiomethyl-6-äthyl-, 2-tert.-Butylthiomethyl-6-äthyl-, 2-Monomethylaminomethyl-6-äthyl-, 2-Monoäthylaminomethyl-6-äthyl-, 2-Mono-n-propylaminomethyl-6-äthyl-, 2-Mono-iso-propylaminomethyl-6-äthyl-, 2-Mono-n-butylaminomethyl-6-äthyl-, 2-Mono-iso-butylaminomethyl-6-äthyl-, 2-Mono-sek.-butylaminomethyl-6-äthyl-, 2-Mono-tert.-butylaminomethyl-6-äthyl-, 2-Dimethylaminomethyl-6-äthyl-, 2-Diäthylaminomethyl-6-äthyl-, 2-Methoxymethyl-5-chlor-6-äthyl-, 2-Äthoxymethyl-5-chlor-6-äthyl-, 2-n-Propoxymethyl-5-chlor-6-äthyl-, 2-iso-Propoxymethyl-5-chlor-6-äthyl-, 2-n-Butoxymethyl-5-chlor-6-äthyl-, 2-iso-Butoxymethyl-5-chlor-6-äthyl-, 2-sek.-Butoxymethyl-5-chlor-6-äthyl-, 2-tert.-Butoxymethyl-5-chlor-6-äthyl-, 2-Methylthiomethyl-5-chlor-6-äthyl-, 2-Äthylthiomethyl-5-chlor-6-äthyl-, 2-n-Propylthiomethyl-5-chlor-6-äthyl-, 2-iso-Propylthiomethyl-5-chlor-6-äthyl-, 2-n-Butylthiomethyl-5-chlor-6-äthyl-, 2-iso-Butylthiomethyl-5-chlor-6-äthyl-, 2-sek.-Butylthiomethyl-5-chlor-6-äthyl-, 2-tert.-Butylthiomethyl-5-chlor-6-äthyl-, 2-Monomethylaminomethyl-5-chlor-6-äthyl-, 2-Monoäthylaminomethyl-5-chlor-6-äthyl-, 2-Mono-n-propylaminomethyl-5-chlor-6-äthyl-, 2-Mono-iso-propylaminomethyl-5-chlor-6-äthyl-, 2-Mono-n-butylaminomethyl-5-chlor-6-äthyl-, 2-Mono-iso-butylaminomethyl-5-chlor-6-äthyl-, 2-Mono-sek.-butylaminomethyl-5-chlor-6-äthyl-, 2-Mono-tert.-butylaminomethyl-5-chlor-6-äthyl-, 2-Dimethylaminomethyl-5-chlor-6-äthyl-, 2-Methoxymethyl-5-brom-6-äthyl-, 2-Äthoxymethyl-5-brom-6-äthyl-, 2-n-Propoxymethyl-5-brom-6-äthyl-, 2-iso-Propoxymethyl-5-brom-6-äthyl-, 2-n-Butoxymethyl-5-brom-6-äthyl-, 2-iso-Butoxymethyl-5-brom-6-äthyl-, 2-sek.-Butoxymethyl-5-brom-6-äthyl-, 2-tert.-Butoxymethyl-5-brom-6-äthyl-, 2-Methylthiomethyl-5-brom-6-äthyl-, 2-Äthylthiomethyl-5-brom-6-äthyl-, 2-n-Propylthiomethyl-5-brom-6-äthyl-, 2-iso-Propylthiomethyl-5-brom-6-äthyl-, 2-n-Butylthiomethyl-5-brom-6-äthyl-, 2-iso-Butylthiomethyl-5-brom-6-äthyl-, 2-sek.-Butylthiomethyl-5-brom-6-äthyl-, 2-tert.-Butylthiomethyl-5-brom-6-äthyl-,

2-Monomethylaminomethyl-5-brom-6-äthyl-, 2-Monoäthylaminomethyl-5-brom-6-äthyl-, 2-Mono-n-propylaminomethyl-5-brom-6-äthyl-, 2-Mono-iso-propylaminomethyl-5-brom-6-äthyl-, 2-Mono-n-butylaminomethyl-5-brom-6-äthyl-, 2-Mono-iso-butylaminomethyl-5-brom-6-äthyl-, 2-Mono-sek.-butylaminomethyl-5-brom-6-äthyl-, 2-Mono-tert.-butylaminomethyl-5-brom-6-äthyl-, 2-Dimethylaminomethyl-5-brom-6-äthyl-, 2-Diäthylaminomethyl-5-brom-6-äthyl-, 2-Methoxymethyl-5-methyl-6-äthyl-, 2-Äthoxymethyl-5-methyl-6-äthyl-, 2-n-Propoxymethyl-5-methyl-6-äthyl-, 2-iso-Propoxymethyl-5-methyl-6-äthyl-, 2-n-Butoxymethyl-5-methyl-6-äthyl-, 2-iso-Butoxymethyl-5-methyl-6-äthyl-, 2-sek.-Butoxymethyl-5-methyl-6-äthyl-, 2-tert -Butoxymethyl-5-methyl-6-äthyl-, 2-Methylthiomethyl-5-methyl-6-äthyl-, 2-Äthylthiomethyl-5-methyl-6-äthyl-, 2-n-Propylthiomethyl-5-methyl-6-äthyl-, 2-iso-Propylthiomethyl-5-methyl-6-äthyl-, 2-n-Butylthiomethyl-5-methyl-6-äthyl-, 2-iso-Butylthiomethyl-5-methyl-6-äthyl-, 2-sek.-Butylthiomethyl-5-methyl-6-äthyl-, 2-tert.-Butylthiomethyl-5-methyl-6-äthyl-, 2-Monomethylaminomethyl-5-methyl-6-äthyl-, 2-Monoäthylaminomethyl-5-methyl-6-äthyl-, 2-Mono-n-propylaminomethyl-5-methyl-6-äthyl-, 2-Mono-iso-propylaminomethyl-5-methyl-6-äthyl-, 2-Mono-n-butylaminomethyl-5-methyl-6-äthyl-, 2-Mono-iso-butylaminomethyl-5-methyl-6-äthyl-, 2-Mono-sek.-butylaminomethyl-5-methyl-6-äthyl-, 2-Mono-tert.-butylaminomethyl-5-methyl-6-äthyl-, 2-Dimethylaminomethyl-5-methyl-6-äthyl-, 2-Diäthylaminomethyl-5-methyl-6-äthyl-4-hydroxypyrimidin.

Weiterhin werden als Ausgangsstoffe die 2-Halogenmethyl-pyrimidin(4)yl(thiono)(thiol)-phosphor(phosphon)-säureester bzw. -esteramide (IV) verwendet, die zum Teil bekannt sind (vergleiche z.B. Belgische Patentschrift 636 5o9) oder nach literaturbekannten Verfahren herstellbar sind.

Als Beispiele dafür seien im einzelnen genannt:

O,O-Dimethyl-, O,O-Diäthyl-, O,O-Di-n-propyl-, O,O-Di-iso-propyl-, O-Methyl-O-äthyl-, O-Methyl-O-n-propyl-, O-Methyl-O-iso-propyl-, O-Methyl-O-n-butyl-, O-Methyl-O-iso-butyl-, O-Methyl-O-sek.-butyl-, O-Äthyl-O-n-propyl-, O-Äthyl-O-iso-propyl-, O-Äthyl-O-n-butyl-, O-Äthyl-O-sek.-butyl-, O-Äthyl-O-iso-butyl-, O-n-Propyl-O-butyl- bzw. O-iso-Propyl-O-butyl-O-[2-halogenmethyl-pyrimidin(4)yl]-phosphorsäureester, O,O-Dimethyl-, O,O-Diäthyl-, O,O-Di-n-propyl-, O,O-Di-iso-propyl-, O-Methyl-O-äthyl-, O-Methyl-O-n-propyl-, O-Methyl-O-iso-propyl-, O-Methyl-O-n-butyl-, O-Methyl-O-iso-butyl-, O-Methyl-O-sek.-butyl-, O-Äthyl-O-n-propyl-, O-Äthyl-O-iso-propyl-, O-Äthyl-O-n-butyl-, O-Äthyl-O-sek.-butyl-, O-Äthyl-O-iso-butyl-, O-n-Propyl-O-butyl- bzw. O-iso-Propyl-O-butyl-O-[2-halogenmethyl-5-chlor-pyrimidin(4)yl]-phosphorsäureester, O,O-Dimethyl-, O,O-Diäthyl-, O,O-Di-n-propyl-, O,O-Di-iso-propyl-, O-Methyl-O-äthyl-, O-Methyl-O-n-propyl-, O-Methyl-O-iso-propyl-, O-Methyl-O-n-butyl-, O-Methyl-O-iso-butyl-, O-Methyl-O-sek.-butyl-, O-Äthyl-O-n-propyl-, O-Äthyl-O-iso-propyl-, O-Äthyl-O-n-butyl-, O-Äthyl-O-sek.-butyl-, O-Äthyl-O-iso-butyl-, O-n-Propyl-O-butyl- bzw. O-iso-Propyl-O-butyl-O-[2-halogenmethyl-5-brom-pyrimidin(4)yl]-phosphorsäureester, O,O-Dimethyl-, O,O-Diäthyl-, O,O-Di-n-propyl-, O,O-Di-iso-propyl-, O-Methyl-O-äthyl-, O-Methyl-O-n-propyl-, O-Methyl-O-iso-propyl-, O-Methyl-O-n-butyl-, O-Methyl-O-iso-butyl-, O-Methyl-O-sek.-butyl-, O-Äthyl-O-n-propyl-, O-Äthyl-O-iso-propyl-, O-Äthyl-O-n-butyl-, O-Äthyl-O-sek.-butyl-, O-Äthyl-O-iso-butyl-, O-n-Propyl-O-butyl- bzw. O-iso-Propyl-O-butyl-O-[2-halogenmethyl-5-methyl-pyrimidin(4)yl]-phosphorsäureester, O,O-Dimethyl-, O,O-Diäthyl-, O,O-Di-n-propyl-, O,O-Di-iso-propyl-, O-Methyl-O-äthyl-, O-Methyl-O-n-propyl-, O-Methyl-O-iso-propyl-, O-Methyl-O-n-butyl-, O-Methyl-O-iso-butyl-, O-Methyl-O-sek.-butyl-, O-Äthyl-O-n-propyl-, O-Äthyl-O-iso-propyl-, O-Äthyl-O-n-butyl-, O-Äthyl-O-sek.-butyl-, O-Äthyl-O-iso-butyl-, O-n-Propyl-O-butyl- bzw. O-iso-Propyl-O-butyl-O-[2-halogenmethyl-6-methyl-pyrimidin(4)yl]-phosphorsäureester, O,O-Dimethyl-, O,O-Diäthyl-, O,O-Di-n-propyl-, O,O-Di-iso-propyl-, O-Methyl-O-äthyl-, O-Methyl-O-n-propyl-, O-Methyl-O-iso-propyl-, O-Methyl-O-n-butyl-, O-Methyl-O-iso-butyl-, O-Methyl-O-sek.-butyl-, O-Äthyl-O-n-propyl-, O-Äthyl-O-iso-propyl-, O-Äthyl-O-n-butyl-, O-Äthyl-O-sek.-butyl-, O-Äthyl-O-iso-butyl-, O-n-Propyl-O-butyl- bzw. O-iso-Propyl-O-butyl-O-[2-halogenmethyl-6-äthyl-pyrimidin(4)yl]-phosphorsäureester, O,O-Dimethyl-, O,O-Diäthyl-, O,O-Di-n-propyl-, O,O-Di-iso-propyl-, O-Methyl-O-äthyl-, O-Methyl-O-n-propyl-, O-Methyl-O-iso-propyl-, O-Methyl-O-n-butyl-, O-Methyl-O-iso-butyl-, O-Methyl-O-sek.-butyl-, O-Äthyl-O-n-propyl-, O-Äthyl-O-iso-propyl-, O-Äthyl-O-n-butyl-, O-Äthyl-O-sek.-butyl-, O-Äthyl-O-iso-butyl-, O-n-Propyl-O-butyl- bzw. O-iso-Propyl-O-butyl-O-[2-halogenmethyl-5-chlor-6-methyl-pyrimidin(4)yl]-phosphorsäureester, O,O-Dimethyl-, O,O-Diäthyl-, O,O-Di-n-propyl-, O,O-Di-iso-propyl-, O-Methyl-O-äthyl-, O-Methyl-O-n-propyl-, O-Methyl-O-iso-propyl-, O-Methyl-O-n-butyl-, O-Methyl-O-iso-butyl-, O-Methyl-O-sek.-butyl-, O-Äthyl-O-n-propyl-, O-Äthyl-O-iso-propyl-, O-Äthyl-O-n-butyl-, O-Äthyl-O-sek.-butyl-, O-Äthyl-O-iso-butyl-, O-n-Propyl-O-butyl- bzw. O-iso-Propyl-O-butyl-O-[2-halogenmethyl-5-brom-6-methyl-pyrimidin(4)yl]-phosphorsäureester, O,O-Dimethyl-, O,O-Diäthyl -, O,O-Di-n-propyl-, O,O-Di-iso-propyl-, O-Methyl-O-äthyl-, O-Methyl-O-n-propyl-, O-Methyl-O-iso-propyl-, O-Methyl-O-n-butyl-, O-Methyl-O-iso-butyl-, O-Methyl-O-sek.-butyl-, O-Äthyl-O-n-propyl-, O-Äthyl-O-iso-propyl-, O-Äthyl-O-n-butyl-, O-Äthyl-O-sek.-butyl-, O-Äthyl-O-iso-butyl-, O-n-Propyl-O-butyl- bzw. O-iso-Propyl-O-butyl-O-[2-halogenmethyl-5,6-dimethyl-pyrimidin(4)yl]-phosphorsäureester, O,O-Dimethyl-, O,O-Diäthyl-, O,O-Di-n-propyl-, O,O-Di-isopropyl-, O-Methyl-O-äthyl-, O-Methyl-O-n-propyl-, O-Methyl-O-iso-propyl-, O-Methyl-O-n-butyl-, O-Methyl-O-iso-butyl-, O-Methyl-O-sek.-butyl-, O-Äthyl-O-n-propyl-, O-Äthyl-O-iso-propyl-, O-Äthyl-O-n-butyl-, O-Äthyl-O-sek.-butyl-,

O-Äthyl-O-iso-butyl-, O-n-Propyl-O-butyl- bzw. O-iso-Propyl-O-butyl-O-[2-halogenmethyl-6-äthyl-pyrimidin(4)yl]-phosphorsäureester, O,O-Dimethyl-, O,O-Diäthyl-, O,O-Di-n-propyl-, O,O-Di-iso-propyl-, O-Methyl-O-äthyl-, O-Methyl-O-n-propyl-, O-Methyl-O-iso-propyl-, O-Methyl-O-n-butyl-, O-Methyl-O-iso-butyl-, O-Methyl-O-sek.-butyl-, O-Äthyl-O-n-propyl-, O-Äthyl-O-iso-propyl-, O-Äthyl-O-n-butyl-, O-Äthyl-O-sek.-butyl-, O-Äthyl-O-iso-butyl-, O-n-Propyl-O-butyl- bzw. O-iso-Propyl-O-butyl-O-[2-halogenmethyl-5-chlor-6-äthyl-pyrimidin(4)yl]-phosphorsäureester, O,O-Dimethyl-, O,O-Diäthyl-, O,O-Di-n-propyl-, O,O-Di-iso-propyl-, O-Methyl-O-äthyl-, O-Methyl-O-n-propyl-, O-Methyl-O-iso-propyl-, O-Methyl-O-n-butyl-, O-Methyl-O-iso-butyl-, O-Methyl-O-sek.-butyl-, O-Äthyl-O-n-propyl-, O-Äthyl-O-iso-propyl-, O-Äthyl-O-n-butyl-, O-Äthyl-O-sek.-butyl-, O-Äthyl-O-iso-butyl-, O-n-Propyl-O-butyl- bzw. O-iso-Propyl-O-butyl-O-[2-halogenmethyl-5-brom-6-äthyl-pyrimidin(4)yl]-phosphorsäureester, O,O-Dimethyl-, O,O-Diäthyl-, O,O-Di-n-propyl-, O,O-Di-iso-propyl-, O-Methyl-O-äthyl-, O-Methyl-O-n-propyl-, O-Methyl-O-iso-propyl-, O-Methyl-O-n-butyl-, O-Methyl-O-iso-butyl-, O-Methyl-O-sek.-butyl-, O-Äthyl-O-n-propyl-, O-Äthyl-O-iso-propyl-, O-Äthyl-O-n-butyl-, O-Äthyl-O-sek.-butyl-, O-Äthyl-O-iso-butyl-, O-n-Propyl-O-butyl- bzw. O-iso-Propyl-O-butyl-O-[2-halogenmethyl-5-methyl-6-äthyl-pyrimidin(4)yl]-phosphorsäureester und jeweils die entsprechenden Thionoanalogen, ferner

O,S-Dimethyl-, O,S-Diäthyl-, O,S-Di-n-propyl-, O,S-Di-iso-propyl-, O,S-Di-n-butyl-, O,S-Di-iso-butyl-, O,S-Di-sek.-butyl-, O-Äthyl-S-n-propyl-, O-Äthyl-S-iso-propyl-, O-Äthyl-S-n-butyl-, O-Äthyl-S-sek.-butyl-, O-n-Propyl-S-äthyl-, O-n-Propyl-S-iso-propyl-, O-n-Butyl-S-n-propyl- und O-sek.-Butyl-S-äthyl-O-[2-halogenmethyl-pyrimidin(4)yl]-thiolophosphorsäureester, O,S-Dimethyl-, O,S-Diäthyl-, O,S-Di-n-propyl-, O,S-Di-iso-propyl-, O,S-Di-n-butyl-, O,S-Di-iso-butyl-, O,S-Di-sek.-butyl-, O-Äthyl-S-n-propyl-, O-Äthyl-S-iso-propyl-, O-Äthyl-S-n-butyl-, O-Äthyl-S-sek.-butyl-, O-n-Propyl-S-äthyl-, O-n-Propyl-S-iso-propyl-, O-n-Butyl-S-n-propyl- und O-sek.-Butyl-S-äthyl-O-[2-halogenmethyl-5-chlor-pyrimidin(4)yl]-thiolophosphorsäureester, O,S-Dimethyl-, O,S-Diäthyl-, O,S-Di-n-propyl-, O,S-Di-iso-propyl-, O,S-Di-n-butyl-, O,S-Di-iso-butyl-, O,S-Di-sek.-butyl-, O-Äthyl-S-n-propyl-, O-Äthyl-S-iso-propyl-, O-Äthyl-S-n-butyl-, O-Äthyl-S-sek.-butyl-, O-n-Propyl-S-äthyl-, O-n-Propyl-S-iso-propyl-, O-n-Butyl-S-n-propyl- und O-sek.-Butyl-S-äthyl-O-[2-halogenmethyl-5-brom-pyrimidin(4)yl]-thiolophosphorsäureester, O,S-Dimethyl-, O,S-Diäthyl-, O,S-Di-n-propyl-, O,S-Di-iso-propyl-, O,S-Di-n-butyl-, O,S-Di-iso-butyl-, O,S-Di-sek.-butyl-, O-Äthyl-S-n-propyl-, O-Äthyl-S-iso-propyl-, O-Äthyl-S-n-butyl-, O-Äthyl-S-sek.-butyl-, O-n-Propyl-S-äthyl-, O-n-Propyl-S-iso-propyl-, O-n-Butyl-S-n-propyl- und O-sek.-Butyl-S-äthyl-O-[2-halogen-methyl-5-methyl-pyrimidin(4)yl]-thiolophosphorsäureester, O,S-Dimethyl-, O,S-Diäthyl-, O,S-Di-n-propyl-, O,S-Di-iso-propyl-, O,S-Di-n-butyl-, O,S-Di-iso-butyl-, O,S-Di-sek.-butyl-, O-Äthyl-S-n-propyl-, O-Äthyl-S-iso-propyl-, O-Äthyl-S-n-butyl-, O-Äthyl-S-sek.-butyl-, O-n-Propyl-S-äthyl-, O-n-Propyl-S-iso-propyl-, O-n-Butyl-S-n-propyl- und O-sek.-Butyl-S-äthyl-O-[2-halogenmethyl-6-methyl-pyrimidin(4)yl]-thiolophosphorsäureester, O,S-Dimethyl-, O,S-Diäthyl-, O,S-Di-n-propyl-, O,S-Di-iso-propyl-, O,S-Di-n-butyl-, O,S-Di-iso-butyl-, O,S-Di-sek.-butyl-, O-Äthyl-S-n-propyl-, O-Äthyl-S-iso-propyl-, O-Äthyl-S-n-butyl-, O-Äthyl-S-sek.-butyl-, O-n-Propyl-S-äthyl-, O-n-Propyl-S-iso-propyl-, O-n-Butyl-S-n-propyl- und O-sek.-Butyl-S-äthyl-O-[2-halogenmethyl-6-äthyl-pyrimidin(4)yl]-thiolophosphorsäureester, O,S-Dimethyl-, O,S-Diäthyl-, O,S-Di-n-propyl-, O,S-Di-iso-propyl-, O,S-Di-n-butyl-, O,S-Di-iso-butyl-, O,S-Di-sek.-butyl-, O-Äthyl-S-n-propyl-, O-Äthyl-S-iso-propyl-, O-Äthyl-S-n-butyl-, O-Äthyl-S-sek.-butyl-, O-n-Propyl-S-äthyl-, O-n-Propyl-S-iso-propyl-, O-n-Butyl-S-n-propyl- und O-sek.-Butyl-S-äthyl-O-[2-halogenmethyl-5-chlor-6-methyl-pyrimidin(4)yl]-thiolophosphorsäureester, O,S-Dimethyl-, O,S-Diäthyl-, O,S-Di-n-propyl-, O,S-Di-iso-propyl-, O,S-Di-n-butyl-, O,S-Di-iso-butyl-, O,S-Di-sek.-butyl-, O-Äthyl-S-n-propyl-, O-Äthyl-S-iso-propyl-, O-Äthyl-S-n-butyl-, O-Äthyl-S-sek.-butyl-, O-n-Propyl-S-äthyl-, O-n-Propyl-S-iso-propyl-, O-n-Butyl-S-n-propyl- und O-sek.-Butyl-S-äthyl-O-[2-halogenmethyl-5-brom-6-methyl-pyrimidin(4)yl]-thiolophosphorsäureester, O,S-Dimethyl-, O,S-Diäthyl-, O,S-Di-n-propyl-, O,S-Di-iso-propyl-, O,S-Di-n-butyl-, O,S-Di-iso-butyl-, O,S-Di-sek.-butyl-, O-Äthyl-S-n-propyl-, O-Äthyl-S-iso-propyl-, O-Äthyl-S-n-butyl-, O-Äthyl-S-sek.-butyl-, O-n-Propyl-S-äthyl-, O-n-Propyl-S-iso-propyl-, O-n-Butyl-S-n-propyl- und O-sek.-Butyl-S-äthyl-O-[2-halogenmethyl-5,6-dimethyl-pyrimidin(4)yl]-thiolophosphorsäureester, O,S-Dimethyl-, O,S-Diäthyl-, O,S-Di-n-propyl-, O,S-Di-iso-propyl-, O,S-Di-n-butyl-, O,S-Di-iso-butyl-, O,S-Di-sek.-butyl-, O-Äthyl-S-n-propyl-, O-Äthyl-S-iso-propyl-, O-Äthyl-S-n-butyl-, O-Äthyl-S-sek.-butyl-, O-n-Propyl-S-äthyl-, O-n-Propyl-S-iso-propyl-, O-n-Butyl-S-n-propyl- und O-sek.-Butyl-S-äthyl-O-[2-halogenmethyl-6-äthyl-pyrimidin(4)yl]-thiolophosphorsäureester, O,S-Dimethyl-, O,S-Diäthyl-, O,S-Di-n-propyl-, O,S-Di-iso-propyl-, O,S-Di-n-butyl-, O,S-Di-iso-butyl-, O,S-Di-sek.-butyl-, O-Äthyl-S-n-propyl-, O-Äthyl-S-iso-propyl-, O-Äthyl-S-n-butyl-, O-Äthyl-S-sek.-butyl-, O-n-Propyl-S-äthyl-, O-n-Propyl-S-iso-propyl-, O-n-Butyl-S-n-propyl- und O-sek.-Butyl-S-äthyl-O-[2-halogenmethyl-5-chlor-6-äthyl-pyrimidin(4)yl]-thiolophosphorsäureester, O,S-Dimethyl-, O,S-Diäthyl-, O,S-Di-n-propyl-, O,S-Di-iso-propyl-, O,S-Di-n-butyl-, O,S-Di-iso-butyl-, O,S-Di-sek.-butyl-, O-Äthyl-S-n-propyl-, O-Äthyl-S-iso-propyl-, O-Äthyl-S-n-butyl-, O-Äthyl-S-sek.-butyl-, O-n-Propyl-S-äthyl-, O-n-Propyl-S-iso-propyl-, O-n-Butyl-S-n-propyl- und O-sek.-Butyl-S-äthyl-O-[2-halogenmethyl-5-brom-6-äthyl-pyrimidin(4)yl]-thiolophosphorsäureester, O,S-Dimethyl-, O,S-Diäthyl-, O,S-Di-n-propyl-, O,S-Di-iso-propyl-, O,S-Di-n-butyl-, O,S-Di-iso-butyl-, O,S-Di-sek.-butyl-, O-Äthyl-S-n-propyl-, O-Äthyl-S-iso-propyl-, O-Äthyl-S-n-butyl-, O-Äthyl-S-sek.-butyl-, O-n-Propyl-S-äthyl-, O-n-Propyl-S-iso-propyl-, O-n-Butyl-S-n-propyl- und O-sek.-Butyl-S-äthyl-O-[2-halogenmethyl-5-methyl-6-äthyl-pyrimidin(4)yl]-thiolophosphorsäureester und jeweils die entsprechenden Thionoanalogen, ferner O-Methyl-N-methyl-, O-Methyl-N-äthyl-, O-Methyl-N-n-propyl-, O-Methyl-N-iso-propyl-, O-Äthyl-N-methyl-, O-Äthyl-N-äthyl-, O-Äthyl-N-n-propyl-, O-Äthyl-N-iso-propyl-, O-n-Propyl-N-methyl-, O-n-Propyl-N-äthyl-, O-n-Propyl-N-n-propyl-, O-n-Propyl-N-iso-propyl-, O-iso-Propyl-N-methyl-, O-iso-Propyl-N-äthyl-, O-iso-Propyl-N-n-propyl-, O-iso-Propyl-N-iso-propyl-, O-n-Butyl-N-methyl-, O-n-Butyl-N-äthyl-, O-n-Butyl-N-n-propyl-, O-n-Butyl-N-iso-propyl-, O-iso-Butyl-N-methyl-, O-iso-Butyl-N-äthyl-, O-iso-Butyl-N-n-propyl-, O-iso-Butyl-N-iso-propyl-, O-sek.-Butyl-N-methyl-, O-sek.-Butyl-N-äthyl-, O-sek.-Butyl-N-n-propyl- und O-sek.-Butyl-N-iso-propyl-O-[2-halogenmethyl-pyrimidin(4)yl]-phosphorsäurediesteramid, O-Methyl-N-methyl-, O-Methyl-N-äthyl-, O-Methyl-N-n-propyl-, O-Methyl-N-iso-propyl-, O-Äthyl-N-methyl-, O-Äthyl-N-äthyl-, O-Äthyl-N-n-propyl-, O-Äthyl-N-iso-propyl-, O-n-Propyl-N-methyl-, O-n-Propyl-N-äthyl-, O-n-Propyl-N-n-propyl-, O-n-Propyl-N-iso-propyl-, O-iso-Propyl-N-methyl-, O-iso-Propyl-N-äthyl-, O-iso-Propyl-N-n-propyl-, O-iso-Propyl-N-iso-propyl-, O-n-Butyl-N-methyl-, O-n-Butyl-N-äthyl-, O-n-Butyl-N-n-propyl-, O-n-Butyl-N-iso-propyl-, O-iso-Butyl-N-methyl-, O-iso-Butyl-N-äthyl-, O-iso-Butyl-N-n-propyl-, O-iso-Butyl-N-iso-propyl-, O-sek.-Butyl-N-methyl-, O-sek.-Butyl-N-äthyl-, O-sek.-Butyl-N-n-propyl und O-sek.-Butyl-N-iso-propyl-O-[2-halogenmethyl-5-chlor-pyrimidin(4)yl]-phosphorsäurediesteramid, O-Methyl-N-methyl-, O-Methyl-N-äthyl-, O-Methyl-N-n-propyl-, O-Methyl-N-iso-propyl-, O-Äthyl-N-methyl-, O-Äthyl-N-äthyl-, O-Äthyl-N-n-propyl-, O-Äthyl-N-iso-propyl-, O-n-Propyl-N-methyl-, O-n-Propyl-N-äthyl-, O-n-Propyl-N-n-propyl-, O-n-Propyl-N-iso-propyl-,

O-iso-Propyl-N-methyl-, O-iso-Propyl-N-äthyl-, O-iso-Propyl-N-n-propyl-, O-iso-Propyl-N-iso-propyl-, O-n-Butyl-N-methyl-, O-n-Butyl-N-äthyl-, O-n-Butyl-N-n-propyl-, O-n-Butyl-N-iso-propyl-, O-iso-Butyl-N-methyl-, O-iso-Butyl-N-äthyl-, O-iso-Butyl-N-n-propyl-, O-iso-Butyl-N-iso-propyl-, O-sek.-Butyl-N-methyl-, O-sek.-Butyl-N-äthyl-, O-sek.-Butyl-N-n-propyl- und O-sek.-Butyl-N-iso-propyl-O-[2-halogenmethyl-5-brom-pyrimidin-(4)yl]-phosphorsäurediesteramid, O-Methyl-N-methyl-, O-Methyl-N-äthyl-, O-Methyl-N-n-propyl-, O-Methyl-N-iso-propyl-, O-Äthyl-N-methyl-, O-Äthyl-N-äthyl-, O-Äthyl-N-n-propyl-, O-Äthyl-N-iso-propyl-, O-n-Propyl-N-methyl-, O-n-Propyl-N-äthyl-, O-n-Propyl-N-n-propyl-, O-n-Propyl-N-iso-propyl-, O-iso-Propyl-N-methyl-, O-iso-Propyl-N-äthyl-, O-iso-Propyl-N-n-propyl-, O-iso-Propyl-N-iso-propyl-, O-n-Butyl-N-methyl-, O-n-Butyl-N-äthyl-, O-n-Butyl-N-n-propyl-, O-n-Butyl-N-iso-propyl-, O-iso-Butyl-N-methyl-, O-iso-Butyl-N-äthyl-, O-iso-Butyl-N-n-propyl-, O-iso-Butyl-N-iso-propyl-, O-sek.-Butyl-N-methyl-, O-sek.-Butyl-N-äthyl-, O-sek.-Butyl-N-n-propyl- und O-sek.-Butyl-N-iso-propyl-O-[2-halogenmethyl-5-methyl-pyrimidin(4)yl]-phosphorsäurediesteramid, O-Methyl-N-methyl-, O-Methyl-N-äthyl-, O-Methyl-N-n-propyl-, O-Methyl-N-iso-propyl-, O-Äthyl-N-methyl-, O-Äthyl-N-äthyl-, O-Äthyl-N-n-propyl-, O-Äthyl-N-iso-propyl-, O-n-Propyl-N-methyl-, O-n-Propyl-N-äthyl-, O-n-Propyl-N-n-propyl-, O-n-Propyl-N-iso-propyl-, O-iso-Propyl-N-methyl-, O-iso-Propyl-N-äthyl-, O-iso-Propyl-N-n-propyl-, O-iso-Propyl-N-iso-propyl-, O-n-Butyl-N-methyl-, O-n-Butyl-N-äthyl-, O-n-Butyl-N-n-propyl-, O-n-Butyl-N-iso-propyl-, O-iso-Butyl-N-methyl-, O-iso-Butyl-N-äthyl-, O-iso-Butyl-N-n-propyl-, O-iso-Butyl-N-iso-propyl-, O-sek.-Butyl-N-äthyl-, O-sek.-Butyl-N-n-propyl- und O-sek.-Butyl-N-iso-propyl-O-[2-halogenmethyl-6-methyl-pyrimidin-(4)yl]-phosphorsäurediesteramid, O-Methyl-N-methyl-, O-Methyl-N-äthyl-, O-Methyl-N-n-propyl-, O-Methyl-N-iso-propyl-, O-Äthyl-N-methyl-, O-Äthyl-N-äthyl-, O-Äthyl-N-n-propyl-, O-Äthyl-N-iso-propyl-, O-n-Propyl-N-methyl-, O-n-Propyl-N-äthyl-, O-n-Propyl-N-n-propyl-, O-n-Propyl-N-iso-propyl-, O-iso-Propyl-N-

methyl-, O-iso-Propyl-N-äthyl-, O-iso-Propyl-N-n-propyl-, O-iso-Propyl-N-iso-propyl-, O-n-Butyl-N-methyl-, O-n-Butyl-N-äthyl-, O-n-Butyl-N-n-propyl-, O-n-Butyl-N-iso-propyl-, O-iso-Butyl-N-methyl-, O-iso-Butyl-N-äthyl-, O-iso-Butyl-N-n-propyl-, O-iso-Butyl-N-iso-propyl-, O-sek.-Butyl-N-methyl-, O-sek.-Butyl-N-äthyl-, O-sek.-Butyl-N-n-propyl- und O-sek.-Butyl-N-iso-propyl-O-[2-halogenmethyl-6-äthyl-pyrimidin(4)yl]-phosphorsäurediesteramid, O-Methyl-N-methyl-, O-Methyl-N-äthyl-, O-Methyl-N-n-propyl-, O-Methyl-N-iso-propyl-, O-Äthyl-N-methyl-, O-Äthyl-N-äthyl-, O-Äthyl-N-n-propyl-, O-Äthyl-N-iso-propyl-, O-n-Propyl-N-methyl-, O-n-Propyl-N-äthyl-, O-n-Propyl-N-n-propyl-, O-n-Propyl-N-iso-propyl-, O-iso-Propyl-N-methyl-, O-iso-Propyl-N-äthyl-, O-iso-Propyl-N-n-propyl-, O-iso-Propyl-N-iso-propyl-, O-n-Butyl-N-methyl-, O-n-Butyl-N-äthyl-, O-n-Butyl-N-n-propyl-, O-n-Butyl-N-iso-propyl-, O-iso-Butyl-N-methyl-, O-iso-Butyl-N-äthyl-, O-iso-Butyl-N-n-propyl-, O-iso-Butyl-N-iso-propyl-, O-sek.-Butyl-N-methyl-, O-sek.-Butyl-N-äthyl-, O-sek.-Butyl-N-n-propyl- und O-sek.-Butyl-N-iso-propyl-O-[2-halogenmethyl-5-chlor-6-methyl-pyrimidin(4)yl]-phosphorsäurediesteramid, O-Methyl-N-methyl-, O-Methyl-N-äthyl-, O-Methyl-N-n-propyl-, O-Methyl-N-iso-propyl-, O-Äthyl-N-methyl-, O-Äthyl-N-äthyl-, O-Äthyl-N-n-propyl-, O-Äthyl-N-iso-propyl-, O-n-Propyl-N-methyl-, O-n-Propyl-N-äthyl-, O-n-Propyl-N-n-propyl-, O-n-Propyl-N-iso-propyl-, O-iso-Propyl-N-methyl-, O-iso-Propyl-N-äthyl-, O-iso-Propyl-N-n-propyl-, O-iso-Propyl-N-iso-propyl-, O-n-Butyl-N-methyl-, O-n-Butyl-N-äthyl-, O-n-Butyl-N-n-propyl-, O-n-Butyl-N-iso-propyl-, O-iso-Butyl-N-methyl-, O-iso-Butyl-N-äthyl-, O-iso-Butyl-N-n-propyl-, O-iso-Butyl-N-iso-propyl-, O-sek.-Butyl-N-methyl-, O-sek.-Butyl-N-äthyl-, O-sek.-Butyl-N-n-propyl- und O-sek.-Butyl-N-iso-propyl-O-[2-halogenmethyl-5-brom-6-methyl-pyrimidin(4)yl]-phosphorsäurediesteramid, O-Methyl-N-methyl-, O-Methyl-N-äthyl-, O-Methyl-N-n-propyl-, O-Methyl-N-iso-propyl-, O-Äthyl-N-methyl-, O-Äthyl-N-äthyl-, O-Äthyl-N-n-propyl-, O-Äthyl-N-iso-

propyl-, O-n-Propyl-N-methyl-, O-n-Propyl-N-äthyl-, O-n-Propyl-N-n-propyl-, O-n-Propyl-N-iso-propyl-, O-iso-Propyl-N-methyl-, O-iso-Propyl-N-äthyl-, O-iso-Propyl-N-n-propyl-, O-iso-Propyl-N-iso-propyl-, O-n-Butyl-N-methyl-, O-n-Butyl-N-äthyl-, O-n-Butyl-N-n-propyl-, O-n-Butyl-N-iso-propyl-, O-iso-Butyl-N-methyl-, O-iso-Butyl-N-äthyl-, O-iso-Butyl-N-n-propyl-, O-iso-Butyl-N-iso-propyl-, O-sek.-Butyl-N-methyl-, O-sek.-Butyl-N-äthyl-, O-sek.-Butyl-N-n-propyl- und O-sek.-Butyl-N-iso-propyl-O-[2-halogenmethyl-5,6-dimethyl-pyrimidin(4)yl]-phosphorsäurediesteramid, O-Methyl-N-methyl-, O-Methyl-N-äthyl-, O-Methyl-N-n-propyl-, O-Methyl-N-iso-propyl-, O-Äthyl-N-methyl-, O-Äthyl-N-äthyl-, O-Äthyl-N-n-propyl-, O-Äthyl-N-iso-propyl-, O-n-Propyl-N-methyl-, O-n-Propyl-N-äthyl-, O-n-Propyl-N-n-propyl-, O-n-Propyl-N-iso-propyl-, O-iso-Propyl-N-methyl-, O-iso-Propyl-N-äthyl-, O-iso-Propyl-N-n-propyl-, O-iso-Propyl-N-iso-propyl-, O-n-Butyl-N-methyl-, O-n-Butyl-N-äthyl-, O,-n-Butyl-N-n-propyl-, O-n-Butyl-N-iso-propyl-, O-iso-Butyl-N-methyl-, O-iso-Butyl-N-äthyl-, O-iso-Butyl-N-n-propyl-, O-iso-Butyl-N-iso-propyl-, O-sek.-Butyl-N-methyl-, O-sek.-Butyl-N-äthyl-, O-sek.-Butyl-N-n-propyl- und O-sek.-Butyl-N-iso-propyl-O-[2-halogenmethyl-6-äthyl-pyrimidin(4)yl]-phosphorsäurediesteramid, O-Methyl-N-methyl-, O-Methyl-N-äthyl-, O-Methyl-N-n-propyl-, O-Methyl-N-iso-propyl-, O-Äthyl-N-methyl-, O-Äthyl-N-äthyl-, O-Äthyl-N-n-propyl-, O-Äthyl-N-iso-propyl-, O-n-Propyl-N-methyl-, O-n-Propyl-N-äthyl-, O-n-Propyl-N-n-propyl-, O-n-Propyl-N-iso-propyl-, O-iso-Propyl-N-methyl-, O-iso-Propyl-N-äthyl-, O-iso-Propyl-N-n-propyl-, O-iso-Propyl-N-iso-propyl-, O-n-Butyl-N-methyl-, O-n-Butyl-N-äthyl-, O-n-Butyl-N-n-propyl-, O-n-Butyl-N-iso-propyl-, O-iso-Butyl-N-methyl-, O-iso-Butyl-N-äthyl-, O-iso-Butyl-N-n-propyl-, O-iso-Butyl-N-iso-propyl-, O-sek.-Butyl-N-methyl-, O-sek.-Butyl-N-äthyl-, O-sek.-Butyl-N-n-propyl- und O-sek.-Butyl-N-iso-propyl-O-[2-halogenmethyl-5-chlor-6-äthyl-pyrimidin(4)yl]-phosphorsäurediesteramid,

O-Methyl-N-methyl-, O-Methyl-N-äthyl-, O-Methyl-N-n-propyl-, O-Methyl-N-iso-propyl-, O-Äthyl-N-methyl-, O-Äthyl-N-äthyl-, O-Äthyl-N-n-propyl-, O-Äthyl-N-iso-propyl-, O-n-Propyl-N-methyl-, O-n-Propyl-N-äthyl-, O-n-Propyl-N-n-propyl-, O-n-Propyl-N-iso-propyl-, O-iso-Propyl-N-methyl-, O-iso-Propyl-N-äthyl-, O-iso-Propyl-N-n-propyl-, O-iso-Propyl-N-iso-propyl-, O-n-Butyl-N-methyl-, O-n-Butyl-N-äthyl-, O-n-Butyl-N-n-propyl-, O-n-Butyl-N-iso-propyl-, O-iso-Butyl-N-methyl-, O-iso-Butyl-N-äthyl-, O-iso-Butyl-N-n-propyl-, O-iso-Butyl-N-iso-propyl-, O-sek.-Butyl-N-methyl-, O-sek.-Butyl-N-äthyl-, O-sek.-Butyl-N-n-propyl- und O-sek.-Butyl-N-iso-propyl-O-[2-halogenmethyl-5-brom-6-äthyl-pyrimidin(4)yl]-phosphorsäurediesteramid, O-Methyl-N-methyl-, O-Methyl-N-äthyl-, O-Methyl-N-n-propyl-, O-Methyl-N-iso-propyl-, O-Äthyl-N-methyl-, O-Äthyl-N-äthyl-, O-Äthyl-N-n-propyl-, O-Äthyl-N-iso-propyl-, O-n-Propyl-N-methyl-, O-n-Propyl-N-äthyl-, O-n-Propyl-N-n-propyl-, O-n-Propyl-N-iso-propyl-, O-iso-Propyl-N-methyl-, O-iso-Propyl-N-äthyl-, O-iso-Propyl-N-n-propyl-, O-iso-Propyl-N-iso-propyl-, O-n-Butyl-N-methyl-, O-n-Butyl-N-äthyl-, O-n-Butyl-N-n-propyl-, O-n-Butyl-N-iso-propyl-, O-iso-Butyl-N-methyl-, O-iso-Butyl-N-äthyl-, O-iso-Butyl-N-n-propyl-, O-iso-Butyl-N-iso-propyl-, O-sek.-Butyl-N-methyl-, O-sek.-Butyl-N-äthyl-, O-sek.-Butyl-N-n-propyl- und O-sek.-Butyl-N-iso-propyl-O-[2-halogenmethyl-5-methyl-6-äthyl-pyrimidin(4)yl]-phosphorsäurediesteramid und jeweils die entsprechenden Thionoanalogen, ferner
O-Methyl-, O-Äthyl-, O-n-Propyl-, O-iso-Propyl-, O-n-Butyl-, O-iso-Butyl- und O-sek.-Butylmethan- bzw. -äthan-, -n-propan-, -iso-propan-, -n-butan-, -iso-butan-, -sek.-butan- bzw. -phenyl-O-[2-halogenmethyl-pyrimidin(4)yl]-phosphonsäurediester,
O-Methyl-, O-Äthyl-, O-n-Propyl-, O-iso-Propyl-, O-n-Butyl-, O-iso-Butyl- und O-sek.-Butylmethan-, -äthan-, -n-propan-, -iso-propan-, -n-butan-, -iso-butan-, -sek.-butan- bzw. -phenyl-O-[2-halogenmethyl-5-chlor-pyrimidin(4)yl]-phosphonsäurediester,
O-Methyl-, O-Äthyl-, O-n-Propyl-, O-iso-Propyl-, O-n-Butyl-,

O-iso-Butyl- und O-sek.-Butylmethan-, -äthan-, -n-propan-, -iso-propan-, -n-butan-, -iso-butan-, -sek.-butan- bzw. -phenyl-O-[2-halogenmethyl-5-brom-pyrimidin(4)yl]-phosphonsäurediester, O-Methyl-, O-Äthyl-, O-n-Propyl-, O-iso-Propyl-, O-n-Butyl-, O-iso-Butyl- und O-sek.-Butylmethan-, -äthan-, -n-propan-, -iso-propan-, -n-butan-, -iso-butan-, -sek.-butan- bzw. -phenyl-O-[2-halogenmethyl-5-methyl-pyrimidin(4)yl]-phosphonsäurediester, O-Methyl-, O-Äthyl-, O-n-Propyl-, O-iso-Propyl-, O-n-Butyl-, O-iso-Butyl- und O-sek.-Butylmethan- bzw. -äthan-, -n-propan-, -iso-propan-, -n-butan-, -iso-butan-, -sek.-butan- bzw.-phenyl-O-[2-halogenmethyl-6-methyl-pyrimidin(4)yl]-phosphonsäurediester, O-Methyl-, O-Äthyl-, O-n-Propyl-, O-iso-Propyl-, O-n-Butyl-, O-iso-Butyl- und O-sek.-Butylmethan- bzw. -äthan-, -n-propan-, -iso-propan-, -n-butan-, -iso-butan-, -sek.-butan bzw. -phenyl-O-[2-halogenmethyl-6-äthyl-pyrimidin(4)yl]-phosphonsäurediester, O-Methyl-, O-Äthyl-, O-n-Propyl-, O-iso-Propyl-, O-n-Butyl-, O-iso-Butyl- und O-sek.-Butylmethan- bzw. -äthan-, -n-propan-, -iso-propan-, -n-butan-, -iso-butan-, -sek.-butan bzw. -phenyl-O-[2-halogenmethyl-5-chlor-6-methyl-pyrimidin(4)yl]-phosphonsäurediester, O-Methyl-, O-Äthyl-, O-n-Propyl-, O-iso-Propyl-, O-n-Butyl-, O-iso-Butyl- und O-sek.-Butylmethan- bzw. -äthan-, -n-propan-, -iso-propan-, -n-butan-, -iso-butan-, -sek.-butan- bzw. -phenyl-O-[2-halogenmethyl-5-brom-6-methyl-pyrimidin-(4)yl]-phosphonsäurediester, O-Methyl-, O-Äthyl-, O-n-Propyl-, O-iso-Propyl-, O-n-Butyl-, O-iso-Butyl- und O-sek.-Butylmethan-, -äthan-, -n-propan-, -iso-propan-, -n-butan-, -iso-butan-, -sek.-butan- bzw. -phenyl-O-[2-halogenmethyl-5,6-dimethyl-pyrimidin-(4)yl]-phosphonsäurediester, O-Methyl-, O-Äthyl-, O-n-Propyl-, O-iso-Propyl-, O-n-Butyl-, O-iso-Butyl- und O-sek.-Butylmethan-, bzw -äthan-, -n-propan-, -iso-propan-, -n-butan-, -iso-butan-, -sek.-butan- bzw. -phenyl-O-[2-halogenmethyl-6-äthyl-pyrimidin-(4)yl]-phosphonsäurediester, O-Methyl-, O-Äthyl-, O-n-Propyl-, O-iso-Propyl-, O-n-Butyl-, O-iso-Butyl- und O-sek.-Butylmethan-, -äthan-, -n-propan-, -iso-propan-, -n-butan-, -iso-butan-, -sek.-

butan- bzw. -phenyl-O-[2-halogenmethyl-5-chlor-6-äthyl-pyrimidin(4)yl]-phosphonsäurediester, O-Methyl-, O-Äthyl-, O-n-Propyl-, O-iso-Propyl-, O-n-Butyl-, O-iso-Butyl- und O-sek.-Butylmethan- bzw. -äthan-, -n-propan-, -iso-propan-, -n-butan-, -iso-butan-, -sek.-butan- bzw. -phenyl-O-[2-halogenmethyl-5-brom-6-äthyl-pyrimidin(4)yl]-phosphonsäurediester, O-Methyl-, O-Äthyl-, O-n-Propyl-, O-iso-Propyl-, O-n-Butyl-, O-iso-Butyl und O-sek.-Butylmethan- bzw. -äthan-, -n-propan-, -iso-propan-, -n-butan-, -iso-butan-, -sek.-butan- bzw. -phenyl-O-[2-halogenmethyl-5-methyl-6-äthyl-pyrimidin(4)yl]-phosphonsäurediester und jeweils die entsprechenden Thionoanalogen.

Die weiterhin als Ausgangsstoffe zu verwendenden primären und sekundären Amine (V) sind bekannt. Als Beispiele dafür seien im einzelnen genannt:
Methyl-, Äthyl-,n-Propyl-, iso-Propyl-, n-Butyl-, iso-Butyl-, sek.-Butyl-, tert.-Butyl-, Dimethyl- und Diäthylamin.

Die Verfahren zur Herstellung der erfindungsgemäßen Verbindungen werdenbevorzugt unter Mitverwendung geeigneter Lösungs- oder Verdünnungsmittel durchgeführt. Als solche kommen praktisch alle inerten organischen Solventien infrage. Hierzu gehören insbesondere aliphatische und aromatische, gegebenenfalls chlorierte Kohlenwasserstoffe, wie Benzol, Toluol, Xylol, Benzin, Methylenchlorid, Chloroform, Tetrachlorkohlenstoff, Chlorbenzol, oder Äther, z.B. Diäthyl- und Dibutyläther, Dioxan, ferner Ketone, beispielsweise Aceton, Methyläthyl-, Methylisopropyl- und Methylisobutylketon, außerdem Nitrile, wie Aceto- und Propionitril.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise

Triäthylamin, Trimethylamin, Dimethylanilin, Dimethylbenzylamin und Pyridin.

Die Reaktionstemperatur kann innerhalb eines größeren Bereichs variiert werden. Im allgemeinen arbeitet man bei beiden Verfahrensvarianten zwischen 0 und $100^{o}C$, vorzugsweise bei 35 bis $55^{o}C$.

Die Umsetzung läßt man im allgemeinen bei Normaldruck ablaufen.

Zur Durchführung der Verfahrensvarianten setzt man die Ausgangskomponenten meist in äquivalentem Verhältnis ein. Ein Überschuß der einen oder anderen Reaktionskomponente bringt keine wesentlichen Vorteile. Die Reaktionskomponenten werden meist in einem der oben angeführten Lösungsmittel gegebenenfalls in Gegenwart eines Säureakzeptors zusammengegeben und meist bei erhöhter Temperatur zur Vervollständigung der Reaktion mehrere Stunden gerührt.

Danach gibt man ein organisches Lösungsmittel, z.B. Toluol zu und arbeitet die organische Phase wie üblich durch Waschen, Trocknen und Abdestillieren des Lösungsmittels auf.

Die neuen Verbindungen fallen in Form von Ölen an, die sich zum Teil nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese weise gereinigt werden. Zu ihrer Charakterisierung dient der Brechungsindex.

Wie bereits mehrfach erwähnt, zeichnen sich die erfindungsgemäßen substituierten Pyrimidinyl(thiono)(thiol)phosphor(phosphon)-säureester bzw. -esteramide durch eine hervorragende insektizide und akarizide wirksamkeit aus.

Die Wirkstoffe eignen sich bei guter Pflanzenverträglichkeit und günstiger Warmblütertoxizität zur Bekämpfung von tierischen Schädlingen, insbesondere Insekten und Spinntieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.
Aus der Ordnung der Diplopoda z. B. Blaniulus guttulatus.
Aus der Ordnung der Chilopoda z. B. Geophilus carpophagus, Scutigera spec.
Aus der Ordnung der Symphyla z. B. Scutigerella immaculata.
Aus der Ordnung der Thysanura z. B. Lepisma saccharina.
Aus der Ordnung der Collembola z. B. Onychiurus armatus.
Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z. B. Forficula auricularia.
Aus der Ordnung der Isoptera z. B. Reticulitermes spp..
Aus der Ordnung der Anoplura z. B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae,

Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp..

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z.B. Aëdes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp.,

Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.
Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid,

aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe,

wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Beispiel A

Plutella-Test

Lösungsmittel: 3 Gewichtsteile Aceton
Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der Wirkstoffzubereitung besprüht man Kohlblätter (Brassica oleracea) taufeucht und besetzt sie mit Raupen der Kohlschabe (Plutella maculipennis).

Die Abtötung wird in % bestimmt. Dabei bedeutet 100 %, daß alle Raupen abgetötet wurden; 0 % bedeutet, daß keine Raupen abgetötet wurden.

Bei diesem Test zeigen die Wirkstoffe der Beispiele 1, 2, 3, 4, 5, 7, 9, 10, 14, 15, 16, 17, 18, 19, 20, 21, 24, 32, 33, 34, 35 und 36 eine bessere Wirkung als der Stand der Technik.

Beispiel B

Tetranychus-Test (resistent)

Lösungsmittel: 3 Gewichtsteile
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der Wirkstoffzubereitung werden Bohnenpflanzen (Phaseolus vulgaris), die stark von allen Entwicklungsstadien der gemeinen Spinnmilbe oder Bohnenspinnmilbe (Tetranychus urticae) befallen sind, tropfnaß besprüht.

Die Abtötung wird in % bestimmt. Dabei bedeutet 100 %, daß alle Spinnmilben abgetötet wurden; 0 % bedeutet, daß keine Spinnmilben abgetötet wurden.

Bei diesem Test zeigen die Wirkstoffe der Beispiele 1, 2, 5, 7, 9, 10, 11, 18, 32 und 35 eine bessere Wirkung als der Stand der Technik.

Beispiel C

$LT_{100}$-Test für Dipteren
Testtiere: Aedes aegypti
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man etwa 25 Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen die Wirkstoffe der Beispiele 1, 2, 3, 4, 5, 7, 9, 10, 11, 13, 31, 33, 34, 35 und 36 eine bessere Wirkung als der Stand der Technik.

Herstellungsbeispiele

Beispiel 1:

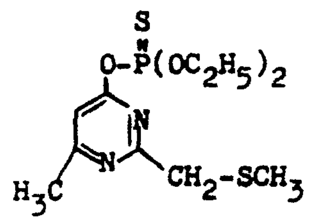

Ein Gemisch aus 3oo ml Acetonitril, 17 g (o,1 Mol) 2-Methylthiomethyl-6-methyl-4-hydroxy-pyrimidin, 2o,7 g (o,15 Mol) Kaliumcarbonat und 18,8 g (o,1 Mol) 0,0-Diäthylthionophosphorsäurediesterchlorid wird 4 Stunden bei 45°C gerührt. Dann gießt man das Reaktionsgemisch in 4oo ml Toluol und wäscht es zweimal mit je 3oo ml Wasser. Die Toluollösung wird über Natriumsulfat getrocknet und im Vakuum eingedampft. Den Rückstand destilliert man im Hochvakuum an. Man erhält so 25 g (78% der Theorie) 0,0-Diäthyl-0-[2-methylthiomethyl-6-methylpyrimidin(4)yl]-thionophosphorsäureester in Form eines braunen Öles mit dem Brechungsindex $n_D^{21}$: 1,5349.

Beispiel 2:

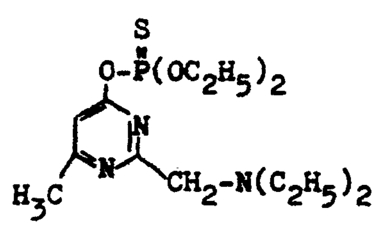

Ein Gemisch aus 3oo ml Acetonitril, 15,9 g (o,1 Mol) 2-Chlormethyl-6-methyl-4-hydroxy-pyrimidin, 48,3 g (o,35 Mol) Kaliumcarbonat und 18,8 g (o,1 Mol) 0,0-Diäthylthionophosphorsäurediesterchlorid wird 3 Stunden bei 45°C gerührt. Danach gibt man bei 0-5°C portionsweise 11 g (o,1 Mol) Diäthylamin-Hydrochlorid zu und rührt das Reaktionsgemisch anschließend 24 Stunden bei

Raumtemperatur nach. Nach Zugabe von 4oo ml Toluol wird der Ansatz zwei mal mit je 3oo ml Wasser gewaschen. Die Toluollösung wird über Natriumsulfat getrocknet und im Vakuum eingedampft. Den Rückstand destilliert man im Hochvakuum an. Man erhält so 31 g (89 % der Theorie) 0,0-Diäthyl-0-[2-diäthylaminomethyl-6-methyl-pyrimidin(4)yl]-thionophosphorsäureester in Form eines braunen Öles mit dem Brechungsindex $n_D^{26}$:1,5o13.

Analog einem der Beispiel 1 oder 2 können die folgenden Verbindungen der Formel

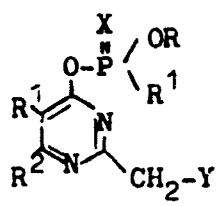

hergestellt werden:

| Beispiel Nr. | R | $R^1$ | $R^2$ | $R^3$ | X | Y | Ausbeute (% der Theorie) | Brechungsindex: |
|---|---|---|---|---|---|---|---|---|
| 3 | $C_2H_5$ | $OC_2H_5$ | $CH_3$ | H | S | $OCH_3$ | 82 | $n_D^{21}$:1,5o34 |
| 4 | $CH_3$ | $OCH_3$ | $CH_3$ | H | S | $OCH_3$ | 65 | $n_D^{21}$:1,5165 |
| 5 | $C_2H_5$ | $CH_3$ | $CH_3$ | H | S | $OCH_3$ | 8o | $n_D^{21}$:1,5218 |
| 6 | $C_2H_5$ | 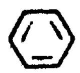 | $CH_3$ | H | S | $OCH_3$ | 83 | $n_D^{22}$:1,5663 |
| 7 | $C_2H_5$ | $SC_3H_7$-n | $CH_3$ | H | S | $OCH_3$ | 8o | $n_D^{22}$:1,5385 |
| 8 | $C_2H_5$ | NH-$C_3H_7$-iso | $CH_3$ | H | S | $OCH_3$ | 69 | $n_D^{22}$:1,5156 |
| 9 | $C_2H_5$ | $OC_2H_5$ | $CH_3$ | H | O | $OCH_3$ | 76 | $n_D^{22}$:1,4827 |
| 1o | $C_2H_5$ | $CH_3$ | $CH_3$ | H | S | $SCH_3$ | 78 | $n_D^{23}$:1,5499 |
| 11 | $CH_3$ | $CH_3$ | $CH_3$ | H | S | $SCH_3$ | 77 | $n_D^{23}$:1,56o2 |
| 12 | $C_2H_5$ | NH-$C_3H_7$-iso | $CH_3$ | H | O | $OCH_3$ | 27 | $n_D^{2o}$:1,4833 |
| 13 | $C_2H_5$ | $OC_2H_5$ | $CH_3$ | H | S | NH-$C_3H_7$-iso | 54 | $n_D^{19}$:1,5o35 |
| 14 | $C_2H_5$ | $OC_2H_5$ | $CH_3$ | H | S | $OC_3H_7$-iso | 66 | $n_D^{26}$:1,4961 |

| Bei-spiel Nr. | R | $R^1$ | $R^2$ | $R^3$ | X | Y | Ausbeute (% der Theorie) | Brechungs-index: |
|---|---|---|---|---|---|---|---|---|
| 15 | $C_2H_5$ | $OC_2H_5$ | $CH_3$ | Br | S | $OCH_3$ | 75 | $n_D^{24}$:1,5272 |
| 16 | $C_2H_5$ | $CH_3$ | $CH_3$ | Br | S | $OCH_3$ | 76 | $n_D^{24}$:1,5422 |
| 17 | $CH_3$ | $OCH_3$ | $CH_3$ | H | S | $OC_3H_7$-iso | 62 | $n_D^{23}$:1,511o |
| 18 | $C_2H_5$ | $CH_3$ | $CH_3$ | H | S | $OC_3H_7$-iso | 85 | $n_D^{23}$:1,51o8 |
| 19 | $C_2H_5$ | $OC_2H_5$ | $CH_3$ | Cl | S | $OCH_3$ | 41 | $n_D^{23}$:1,5163 |
| 2o | $C_2H_5$ | $CH_3$ | $CH_3$ | Cl | S | $OCH_3$ | 74 | $n_D^{23}$:1,5294 |
| 21 | $C_2H_5$ | $OC_2H_5$ | $CH_3$ | H | S | $SC_3H_7$-n | 86 | $n_D^{24}$:1,5229 |
| 22 | $C_2H_5$ | $OC_2H_5$ | $CH_3$ | Br | S | $SCH_3$ | 65 | $n_D^{22}$:1,5575 |
| 23 | $C_2H_5$ | $OC_2H_5$ | $CH_3$ | $CH_3$ | S | $OCH_3$ | 84 | $n_D^{23}$:1,5o68 |
| 24 | $C_2H_5$ | $OC_2H_5$ | $CH_3$ | Cl | S | $OC_3H_7$-iso | 52 | $n_D^{23}$:1,5oo2 |
| 25 | $C_2H_5$ | $OC_2H_5$ | $CH_3$ | Cl | S | $N(C_2H_5)_2$ | | |
| 26 | $C_2H_5$ | $OC_2H_5$ | $CH_3$ | Br | S | $N(C_2H_5)_2$ | | |

| Beispiel Nr. | R | $R^1$ | $R^2$ | $R^3$ | X | Y | Ausbeute (% der Theorie) | Brechungsindex: |
|---|---|---|---|---|---|---|---|---|
| 27 | $C_2H_5$ | $OC_2H_5$ | $CH_3$ | $CH_3$ | S | $N(C_2H_5)_2$ | | |
| 28 | $C_2H_5$ | $OC_2H_5$ | $CH_3$ | Cl | S | $NH-C_3H_7$-iso | | |
| 29 | $C_2H_5$ | $OC_2H_5$ | $CH_3$ | Br | S | $NH-C_3H_7$-iso | | |
| 3o | $C_2H_5$ | $OC_2H_5$ | $CH_3$ | $CH_3$ | S | $NH-C_3H_7$-iso | | |
| 31 | $CH_3$ | $OCH_3$ | $CH_3$ | H | S | $SC_2H_5$ | 79 | $n_D^{2o}$:1,5312 |
| 32 | $C_2H_5$ | $C_2H_5$ | $CH_3$ | H | S | $SC_2H_5$ | 89 | $n_D^{2o}$:1,533o |
| 33 | $C_2H_5$ | $OC_2H_5$ | $CH_3$ | H | S | $SC_2H_5$ | 9o | $n_D^{2o}$:1,5221 |
| 34 | $CH_3$ | $OCH_3$ | $CH_3$ | H | S | $OC_2H_5$ | 75 | $n_D^{2o}$:1,5o5o |
| 35 | $C_2H_5$ | $C_2H_5$ | $CH_3$ | H | S | $OC_2H_5$ | 8o | $n_D^{2o}$:1,51oo |
| 36 | $C_2H_5$ | $OC_2H_5$ | $CH_3$ | H | S | $OC_2H_5$ | 85 | $n_D^{2o}$:1,4949 |

| Beispiel Nr. | R | $R^1$ | $R^2$ | $R^3$ | X | Y | Ausbeute (% der Theorie) | Brechungsindex: |
|---|---|---|---|---|---|---|---|---|
| 37 | $C_2H_5$ | $OC_2H_5$ | H | H | S | $OCH_3$ | | |
| 38 | $C_2H_5$ | $OC_2H_5$ | H | H | S | $OC_2H_5$ | | |
| 39 | $C_2H_5$ | $OC_2H_5$ | H | H | S | $SCH_3$ | | |
| 4o | $C_3H_7$-iso | $CH_3$ | $CH_3$ | H | S | $OCH_3$ | | |

Die als Ausgangsmaterialien zu verwendenden 4-Hydroxy-pyrimidine können z.B. wie folgt hergestellt werden:

**Beispiel a:**

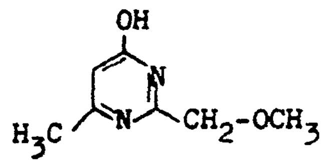

Zu einer Lösung von 8o g (o,5 Mol) 2-Chlormethyl-4-hydroxy-6-methylpyrimidin [Darstellung s. T. Kato, H. Yamanaka und J. Kawamata Yakugaku Zasshi 89, 46o-463(1969); Chemical Abstracts 71, 7o653a (1969)] in 1oo ml Methanol tropft man eine Lösung von 6o g (1,1 Mol) Natriummethylat in 2oo ml Methanol. Das Gemisch wird 2 Stunden bei 55-6o°C gerührt, dann auf Raumtemperatur abgekühlt und mit wäßriger Salzsäure auf pH 5-6 gebracht. Nach Abdestillieren des Lösungsmittels trocknet man den Rückstand bei 7o°C, verreibt ihn mit 8oo ml warmem Aceton, filtriert vom Ungelösten und dampft das Filtrat im Vakuum zur Trockne ein. Zurück bleiben 6o g (78 % der Theorie) 2-Methoxymethyl-4-hydroxy-6-methylpyrimidin in Form farbloser Kristalle mit dem Schmelzpunkt 1o1°C.

**Beispiel b:**

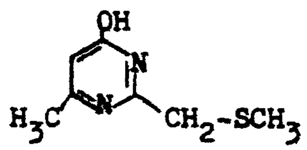

Ein Gemisch aus 119 g (o,75 Mol) 2-Chlormethyl-4-hydroxy-6-methylpyrimidin, 52,5 g (o,75 Mol) Natriummethylmercaptid und 7oo ml Acetonitril wird 3 Stunden unter Rückfluß gekocht. Dann filtriert man das heiße Reaktionsgemisch und dampft das Filtrat im Vakuum zur Trockne ein. Man erhält so 68 g (53 % der Theorie) 2-Methylthiomethyl-4-hydroxy-6-methylpyrimidin in Form eines beigen Pulvers mit dem Schmelzpunkt 138°C.

Analog einem der Beispiele a oder b können die folgenden Verbindungen der Formel

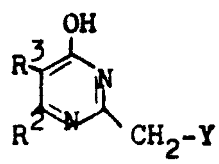

hergestellt werden:

| Beispiel | $R^2$ | $R^3$ | Y | Ausbeute (% der Theorie) | Schmelzpunkt °C |
|---|---|---|---|---|---|
| c | $CH_3$ | H | $OC_3H_7$-iso | 67 | 61 |
| | $CH_3$ | H | $SC_3H_7$-n | 86 | 87 |
| e | $CH_3$ | Cl | $OCH_3$ | 88 | 142 |
| f | $CH_3$ | Cl | $OC_3H_7$-iso | 51 | 206(Z) |
| g | $CH_3$ | Br | $OCH_3$ | 97 | 148 |
| h | $CH_3$ | Cl | $SCH_3$ | 56 | 165(Z) |
| i | $CH_3$ | $CH_3$ | $SCH_3$ | 33 | 142 |
| j | $CH_3$ | Br | $SCH_3$ | 58 | 137 |
| k | $CH_3$ | $CH_3$ | $OCH_3$ | 89 | 112 |
| l | $CH_3$ | H | $OC_2H_5$ | 84 | 68 |

| Beispiel | $R^2$ | $R^3$ | Y | Ausbeute (% der Theorie) | Schmelzpunkt °C |
|---|---|---|---|---|---|
| m | $CH_3$ | H | $SC_2H_5$ | 56 | 132 |
| n | H | H | $OCH_3$ | | |
| o | H | H | $OC_2H_5$ | | |
| p | H | H | $SCH_3$ | | |
| q | $CH_3$ | H | $N(CH_3)_2$ | 92 | 161 |

Patentansprüche

1. Pyrimidinyl(thiono)-(thiol)-phosphor(phosphon)-säureester bzw. -esteramide der Formel (I)

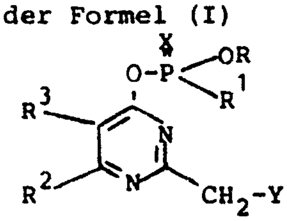

(I)

in welcher

R für Alkyl
$R^1$ für Alkyl, Phenyl, Alkoxy, Alkylthio oder Alkylamino,
$R^2$ und $R^3$ gleich oder verschieden sind und für Wasserstoff, Halogen oder Alkyl,
Y für Alkoxy, Alkylthio oder Mono- bzw. Dialkylamino und
X für Sauerstoff oder Schwefel stehen.

2. Verfahren zur Herstellung der Pyrimidinyl-(thiono)(thiol)-phosphor(phosphon)-säureester und -esteramide der Formel (I) gemäß Anspruch 1, dadurch gekennzeichnet, daß man

a) (Thiono)(thiol)phosphor(phosphon)-säureester- bzw. -esteramidhalogenide der Formel (II)

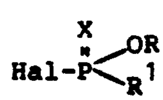

(II)

in welcher

R, $R^1$ und X die in Anspruch 1 angegebene Bedeutung haben und

Hal für Halogen, vorzugsweise Chlor steht, mit substituierten 4-Hydroxy-pyrimidinen der Formel (III)

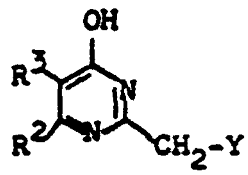

(III)

in welcher

$R^2$, $R^3$ und Y die in Anspruch 1 angegebene Bedeutung haben, gegebenenfalls in Gegenwart eines Säureakzeptors oder gegebenenfalls in Form der Alkali-, Erdalkali- oder Ammoniumsalze und gegebenenfalls in Gegenwart eines Lösungsmittels umsetzt, oder für den Fall, daß Y für Mono- bzw. Dialkylamino steht,

b) 2-Halogenmethyl-pyrimidin(4)yl(thiono)(thiol)-phosphor-(phosphon)-säureester bzw. -esteramide der Formel (IV)

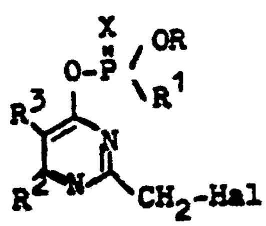

(IV)

in welcher

R bis $R^3$, X und Hal die in Anspruch 1 angegebene Bedeutung haben,

mit Verbindungen der Formel (V)

Y'H (V)

in welcher

Y' für Mono- oder Dialkylamino steht,

gegebenenfalls in Gegenwart eines Säureakzeptors und gegebenenfalls in Gegenwart eines Lösungsmittels umsetzt.

3. Insektizide und akarizide Mittel gekennzeichnet durch einen Gehalt an mindestens einem Pyrimidinyl(thiono) (thiol)-phosphor(phosphon)-säureester bzw. -esteramid der Formel (I) gemäß Anspruch 1.

4. Verwendung von Pyrimidinyl(thiono)(thiol)-phosphor(phosphon)-säureestern bzw. -esteramiden gemäß Anspruch 1 zur Bekämpfung von Insekten oder Acarinae.

5. Verfahren zur Bekämpfung von Insekten oder Acarinae, dadurch gekennzeichnet, daß man Pyrimidinyl(thiono) (thiol)-phosphor(phosphon)-säureester bzw. -esteramid gemäß Anspruch 1 auf Insekten oder Acarinae und/oder auf ihren Lebensraum einwirken läßt.

6. Verfahren zur Herstellung insektizider oder akarizider Mittel, dadurch gekennzeichnet, daß man Pyrimidinyl(thiono)-(thiol)-phosphorsäureester bzw. -esteramide der Formel I gemäß Anspruch 1 mit Streckmitteln und/oder oberflächenaktiven Stoffen vermischt.

0001602

Nummer der Anmeldung

Europäisches Patentamt

# EUROPÄISCHER RECHERCHENBERICHT

EP 78 101 097.0

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| - | CH - A - 498 871 (AGRIPAT)<br>* Anspruch *<br>& US - A - 3 367 935<br>-- | 2 | C 07 F 9/65<br>A 01 N 9/36 |
| D | US - A - 2 754 243 (GEIGY)<br>* Tabelle Nr. 25 bis 29; Anspruch 2 *<br>-- | 1-6 | |
| A | DE - A - 2 343 931 (BAYER)<br>* ganzes Dokument *<br>-- | | RECHERCHIERTE SACHGEBIETE (Int. Cl.²)<br>A 01 N 9/36<br>C 07 F 9/65 |
| A | GB - A - 1 203 026 (ICI)<br>* ganzes Dokument *<br>---- | | |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| Berlin | 22-01-1979 | KAPTEYN |

EPA form 1503.1 06.78